# EUROPÄISCHE PATENTSCHRIFT

(11) **EP 1 391 197 B1**
(45) Veröffentlichungstag und Bekanntmachung des Hinweises auf die Patenterteilung: **03.05.2006**
(21) Anmeldenummer: 03016673.0
(22) Anmeldetag: 01.08.2003
(51) Int. Cl.: A61K 31/05, A61K 35/56, A61P 17/00, A61P 35/00, A61P 31/12, A61P 29/00, A61P 19/00

(54) **Verfahren zur Herstellung von Zusammensetzungen mit hohem Avarol - Gehalt sowie deren Verwendung**
Process for preparation of compositions with a high content in avarol and their use
Procédé de préparation de compositions à haute teneur en avarol et leur utilisation

(30) Priorität: 21.08.2002 DE 10238169
(43) Veröffentlichungstag der Anmeldung: 25.02.2004
(73) Patentinhaber: KliniPharm GmbH, 60318 Frankfurt / Main (DE)
(72) Erfinder: Schatton, Wolfgang, Dr., 65760 Eschborn (DE); Schatton, Maria, 65760 Eschborn (DE); Pietschmann, Rainer, Dr., 65719 Hofheim (DE)
(74) Vertreter: Müller, Claudia

(56) Entgegenhaltungen:
- DE-A- 3 621 032
- DATABASE MEDLINE [Online] US NATIONAL LIBRARY OF MEDICINE (NLM), BETHESDA, MD, US; August 2000 (2000-08) MÜLLER W E ET AL: "Application of cell culture for the production of bioactive compounds from sponges: synthesis of avarol by primmorphs from Dysidea avara." Database accession no. NLM10978201 XP002261022 & JOURNAL OF NATURAL PRODUCTS. UNITED STATES AUG 2000, Bd. 63, Nr. 8, August 2000 (2000-08), Seiten 1077-1081, ISSN: 0163-3864
- BELISARIO M A ET AL: "EFFECT OF AVAROL AND AVARONE ON IN-VITRO-INDUCED MICROSOMAL LIPID PEROXIDATION" TOXICOLOGY, Bd. 72, Nr. 2, 1992, Seiten 221-233, XP008024427 ISSN: 0300-483X

## Beschreibung

### Gegenstand der Erfindung

Die vorliegende Erfindung betrifft das in den Ansprüchen beschriebene vereinfachte Verfahren zur Herstellung einer Avarol- Zusammensetzung mit bis zu 98 % Avarol, welche insbesondere noch einen Anteil an insbesondere anti - oxidativen Verbindungen aufweist. Hierzu wird das Avarol - haltige Ausgangsmaterial mit einem polaren Hydroxylgruppen - haltigen Lösungsmittel versetzt und sodann ein Reduktionsmittel wie Alkalisalze von Dithionit-, -Disulfit- , Bisulfit- Verbindungen hinzugefügt. Überraschender weise wird auf diese einfache Weise in hohen Ausbeuten eine Avarol - Zusammensetzung gewonnen, die vorzugsweise 65-95 % Avarol und 5 bis 35 Gew. % an antioxidativen Verbindungen aufweist. Damit ist eine verbesserte Wirkung zur Behandlung, Prävention proliferafiver Funktionsstörungen bei Menschen oder Tieren möglich. Ferner kann durch eine besondere Flüssig-flüssig Extraktion aus dieser Zusammensetzung hochreines Avarol oder durch Umsetzung mit einem Oxidationsmittel wie einer Cer(IV)- Verbindung hochreines Avaron in hohen Mengen gewonnen werden.

### Stand der Technik

Avarol und Avaron sind wichtige Metabolite von Meeresschwämmen wie z. B. des Meeresschwamms Dysidea avara und als solche bekannt, wie beispielsweise in "Drugs of the Future, 10 (1985) 887-889 " beschrieben. Wie hieraus ersichtlich ist, konnten diese Substanzen bisher nur unter sehr hohen Kosten und in langwierigen, zeitaufwendigen chromatographischen Verfahren gewonnen werden. Dabei wurden den Meeresschwämmen zunächst polare Lösungsmittel wie Essigester, Aceton oder Mischungen hiervon zugesetzt und der erhaltene flüssige Extrakt eingeengt. Dabei wurde eine braune ölige Substanz erhalten. Der Grund dafür liegt im wesentlichen darin, dass der Schwamm sich bereits unter Wasser unmittelbar an der Schnittstelle sehr rasch violett verfärbt. Diese Verfärbung schreitet an der Luft, bei der Extraktion und Konzentration so rasch fort, dass schließlich ein schwarzbraun gefärbter, teeriger Schwammextrakt resultiert, der auch in der Literatur an verschiedenen Stellen immer wieder beschrieben wird.
Das erhaltene ölige Produkt muss daher in mehreren Schritten aufwendig gereinigt werden wie wiederholte präparative Säulenchromatographie an normalem oder reversed Phase Kieselgel oder präparative HPLC, mit anschließender wiederholter Umkristallisation. Dabei kann der Verlust hoher Mengen an gewünschtem Produkt durch die erwähnten unvermeidbaren unerwünschten chemischen Nebenreaktionen nicht vermieden werden.
Die US - A 5,082,865 betrifft die Verwendung von reinem Avarol zur Behandlung von Tumoren. Dabei wird die aktive Substanz wie beschrieben aus dem Schwamm Dysidea avara mittels Essigesterextraktion und nachfolgende Chromatographie an Kieselgel in einer Menge von 2,7 g Reinsubstanz aus 1 Kg Ausgangmaterial (frischer Schwamm) erhalten. Avarol kann durch Umsetzung mit Silberoxid AgO₂ in Avaron überführt werden. Beide Wirkstoffe können auf bekannte Wiese in pharmazeutisch verträgliche Derivate wie Ester oder Additionssalze überführt werden, die bei Anwendung die pharmazeutisch wirksame Form freisetzen.
Aus der DE 41 37 093 A1 ist die Verwendung eines auf dieselbe Weise gewonnenen reinen Avarol- Produktes als entzündungshemmendes systemisches oder dermatologisches Mittel insbesondere zur Behandlung atopischer Dermatitis oder rheumatoider Erkrankungen beschrieben. Hier kann der Wirkstoff topisch z. B. in Form einer Salbe, Lotion, Creme, oder auch oral in einer flüssigen Formulierung auf wässriger Basis, als Tablette, oder Kapsel mit üblichen bekannten Zusatzstoffen eingesetzt werden. Hier wird festgestellt, dass eine antioxidative Wirkung des Avarol zur Inhibierung der Enzyme, die an der Synthese von Prostaglandinen und Leukotrienen (Cyclooxygenase- und 5-Lipoxygenase- Inhibitoren) beteiligt sind, vorliegt.

Die US-A 49 39,177 beschreibt die Verwendung von Avarol zur Kontrolle von AIDS. Hier wird Avarol auf die oben beschriebene Weise erhalten.
Im J. Org. Chem. 1982, 47, 1727-31 wird ein vielstufiges Verfahren zur Totalsynthese von Avarol beschrieben, wobei kostenaufwendige Reaktionen durchgeführt werden, da auf die Stereochemie zu achten ist und die Ausbeute der allein der letzten 8 Schritte ohne die ersten Reaktionsstufen maximal 28 % beträgt.

Dokument XP0022610 22 beschreibt die Gewinnnung von reinem Avarol aus Primorph-Kulturen. Die DE 3621 032 beschreibt ein Verfahren zur Hestellung von reinem Avarol aus Meeresschwämmen durch Ethylacetat-Extraktion.

Dokument XP 008024427 offenbart die wirksamkeit von Avarol oder Avaron bei der la vitro induzierten Lipidoxidation.

Für eine dermatologisch / kosmetische oder medizinische Verwendung von Avarol oder Avaron sind die oben genannten Verfahren daher wenig geeignet, da sie zu kosten- und / oder zeitaufwendig sind.

### Aufgabe der Erfindung

Aufgabe vorliegender Erfindung war es daher, ein Verfahren zu entwickeln, bei welchem ohne hohen Kosten- und Zeitaufwand Avarol so gewonnen werden kann, dass es in dermatologisch/ kosmetischen oder pharmazeutischen Anwendungen eingesetzt werden kann. Ferner soll auf einfache Weise auch das pharmazeutisch wirksame Avaron auf einfache Weise erhältlich sein.

### Lösung der Aufgabe und Gegenstand der Erfindung

Diese Aufgabe wird erfindungsgemäß dadurch gelöst, dass man das Avarol - haltige Ausgangsmaterial in einem Hydroxylgruppen - haltigen polaren Lösungsmittel, insbesondere ein- oder mehrwertigen Alkoholen oder Mischungen hiervon oder mit Wasser, umsetzt, sodann ein Reduktionsmittel zusetzt und aus dem so erhaltenen Extrakt Avarol in einer hoch konzentrierten Zusammensetzung erhält. Der Extrakt kann in einem oder mehreren Schritten erneut mit Reduktionsmittel umgesetzt werden, was überraschenderweise zu einer Erhöhung der Reinheit führt.
Dieser Extrakt kann auch in einem weiteren einfachen Schritt zu hoch reinem Avarol oder zu Avaron ohne Einsatz von Silberoxid umgesetzt werden.
Überraschenderweise wird auf diese Weise aus Avarol -haltigern Ausgangsmaterial das gewünschte Produkt in einer Zusammensetzung erhalten, welche noch besser wirksam ist als entzündungshemmendes oder antivirales Mittel als bisher eingesetzte Avarol -Produkte. Darüber hinaus wird das Produkt in sehr hohen Ausbeuten, nämlich weit mehr als das Doppelte bisher erzielter Mengen, auf technisch wenig aufwendigem und vor allem sehr kostengünstigen Weg gewonnen.
Auf die beschriebene Weise können überraschenderweise die unerwünschten Nebenreaktionen, welche durch Reaktion von Avarol / Avaron mit funktionellen Gruppen , z.B. R-NH2, R-SH, R-OH oder anderen, sonstiger vorhandener Begleitstoffe stark gefärbte Reaktionsprodukte ergeben, wirksam unterdrückt werden.

### Nähere Beschreibung der Erfindung

Erfindungsgemäß wird somit ein Verfahren zur Herstellung einer Zusammensetzung, enthaltend Avarol in einer Menge von bis zu 98% , zur Verfügung gestellt, dass dadurch gekennzeichnet ist , dass man ein Avarol - haltiges Ausgangsmaterial ein oder mehrmals in einem polaren Hydroxygruppen - haltigen, insbesondere alkoholischen Lösungsmittel umsetzt und dieser Mischung ein oder mehrere Reduktionsmittel, ausgewählt aus Alkalisalzen von Dithionit-, -disulfit-, bisulfit- Verbindungen, Selenitsalzen, wie z. B. Natriumselenit , Zitronensäure, Ascorbinsäure, Glutathion, Phosphite oder Mischungen hiervon in einer Menge von 0,01 bis 20 Gew. %, bezogen auf die Menge an Ausgangsmaterial, hinzufügt und sodann das im Lösungsmittel vorliegende Produkt gewinnt. Dieses kann z. B. durch Ausfällen z. B. mit Wasser oder wässriger Lösung mit Reduktionsmittel dann in fester Form erhalten werden. Hierbei werden weitaus höhere Ausbeuten als bisher erzielt, z. B. mehr als 1 % pro kg Ausgangsmaterial nasse Schwämme, bzw. mehr als 10 % bei getrockneter Biomasse als Ausgangsmaterial. Im Stand der Technik ist als Ausgangsmaterial keine Biomasse erwähnt und bei nassen Schwämmen werden lediglich 0,27 % (s. o. 2,7 g Avarol aus 1 kg) erhalten.
Als Ausgangsmaterial können vor allem Meeresschwämme, Lösungsmittelextrakte , insbesondere alkoholische Extrakte von Meeresschwämmen oder Schwammzellbiomasse oder gentechnologisch erzeugte Avarol - produzierende Biomasse (wie Algen etc.) eingesetzt werden.

Bevorzugt werden Meeresschwämme oder Schwammbiozellmasse oder gentechnologisch erzeugte Biomasse eingesetzt. Hierbei sind insbesondere Schwämme oder Schwammbiozellmasse bevorzugt. Als Meeresschwämme oder Extrakte hieraus oder Zellbiomasse sind besonders jene der Art Dysidea avara geeignet. Diese sind besonders bevorzugt. Beim besonders bevorzugten Einsatz dieser Meeresschwämme bzw. der Biozellmasse hiervon werden somit Nativ-Extrakte erhalten, welche als solche direkt pharmazeutisch/ dermatologischkosmetisch eingesetzt werden können und insofern dem ursprünglichem Produkt entsprechen.
Schwammzellbiomasse wird vor allem durch Schwammzellkultur oder Schwammgewebekultur in an sich bekannter Weise gewonnen und kann ebenfalls eingesetzt werden.
Als Reduktionsmittel werden insbesondere Natrium- oder Kaliumsalze von Dithionit- (Na₂/K₂- S2O₄), -Disulfit- (Na₂/K₂S₂O₅), Bisulfit-(Na₂/K₂HSO₃) Verbindungen eingesetzt. Ganz besonders bevorzugt sind die Natriumsalze und hierunter vor allem Natriumdithionit.
Die Konzentration an Reduktionsmittel kann variiert werden, je nachdem wie oft der erfindungsgemäße Extraktionsschritt durchgeführt wird. Bevorzugt werden Mengen von 1 bis 15 Gew. %, insbesondere 3 bis 15 Gew. %, vor allem 3 bis 10 Gew.% (bezogen auf die menge an Ausgangsmaterial).
Die Menge an Reduktionsmittel kann auch je nach Ausgangsmaterial variiert werden. So können auch eingeengte Extrakte gemäß Stand der Technik, mit z. B. 0,01- 10 % Reduktionsmittel, insbesondere in Wasser als polares Hydroxylgruppen - haltiges Lösungsmittel behandelt und Avarol extrahiert werden.
Besonders bevorzugt kann auch der auf die geschilderte Weise gewonnene Extrakt erneut, z. B. mit 0,01 bis 3 Gew. % an Reduktionsmittel, insbesondere bevorzugtem Reduktionsmittel , umgesetzt werden, wobei dann dieses im gewählten Lösungsmittel, oder auch in Wasser ,zugesetzt wird und reine Avarol-Zusammensetzungen der beschriebenen Art gewonnen werden.
Werden mehrere Umsetzungen durchgeführt, so ist es bevorzugt, wenn man eine erste Umsetzung mit einer Konzentration an Reduktionsmittel von 3 bis 15 Gew. % vornimmt, und das Lösungsmittel mit der darin enthaltenen Avarol - Zusammensetzung abtrennt, das Ausgangsmaterial erneut ein oder mehrmals mit Reduktionsmittel in einer Menge von jeweils 0,01 bis 3 Gew.% (bezogen auf die Menge an Ausgangsmaterial) mit Lösungsmittel, behandelt und sodann jeweils den Lösungsmittelextrakt mit darin enthaltener Avarol Zusammensetzung vor der folgenden Umsetzung abtrennt und ggf. die gewonnenen Lösungsmittelextrakte vereinigt und die darin enthaltene Avarol - Zusammensetzung gewinnt.
Die Avarol- Zusammensetzung kann insbesondere durch Ausfällen aus den Lösungsmittelextrakt(en) durch ein- oder mehrmalige Zugabe von Wasser oder bevorzugt wässriger Lösung, enthaltend 0,01 bis 10 Gew. %, vor allem 0,01 bis 8, insbesondere 0,01 bis 5 oder bevorzugt 0,01 bis 1 Gew.% Reduktionsmittel, gewonnen werden.
Dabei wird die Avarol - Zusammensetzung in sehr reiner Form gewonnen und weist 60-98 % Avarol und 40 bis 2 Gew.% weitere natürlicher Inhaltsstoffe, insbesondere Antioxidative Substanzen, insbesondere aus der Gruppe der Sesquiterpenderivate auf.
Als polares Hydroxylgruppen - haltiges Lösungsmittel eignen sich neben Wasser insbesondere ein- oder mehrwertige Alkohole oder Mischungen hiervon oder mit Wasser in unterschiedlichen Anteilen wie 99:1 bis 50:50 (Alkohol : Wasser). Als einwertige Alkohole, die besonders bevorzugt sind, sind vor allem C₁₋₆- Alkohole wie Methanol, Ethanol, n-, iso- Propanol, Butanol (n-, iso- oder tert.-), Pentanol (n-, iso-), oder n- Hexanol geignet. Ganz besonders bevorzugt sind Methanol und Ethanol, Propanol, Isopropanol oder Mischungen hiervon oder mit Wasser, z. B. im Verhältnis wie oben angegeben oder 95:5 bis 70:30 (Alkohol : Wasser bzw. Ethanol: Methanol). Besonders bevorzugt sind Ethanol, Methanol, insbesondere Methanol, Mischungen hiervon und mit Wasser.
Als mehrwertige Alkohole eignen sich zwei- und dreiwertige Alkohole mit C₂₋₈₋Kohlenstoffatomen wie Ethylenglykol, Propylenglykol, Butylenglykol, Glycerol oder Mischungen hiervon oder mit Wasser wie angegeben. Ferner kann es auch von Vorteil sein Polyethylenglykol hinzuzufügen zu einem oder mehreren der genannten Alkohole oder Alkohol/Wasser Gemische.
Aromatische substituierte Alkohole sind beispielsweise Benzylalkohol, Thymol; cycloaliphatische Alkohole sind vorzugsweise Cyclohexanol, Cyclopentanol.
Das Lösungsmittel wird in einer Menge von 1 Äquivalent bis 10facher Überschuss, bezogen auf das Gewicht (Menge) an Avarol- haltigem Ausgangsmaterial eingesetzt. Bevorzugt wird die 1,5 bis 8-fache, insbesondere 1,5 bis 5fache Menge eingesetzt.
%- Angaben beziehen sich generell hier auf das Gewicht.

Auf diese Weise wird eine Avarol- haltige Zusammensetzung mit bis zu 98% Avarol erhalten. Die Zusammensetzung weist darüber hinaus noch einen Anteil an zusätzlichen Stoffen, vor allem antioxidativen Verbindungen auf. Insbesondere umfasst die erfindungsgemäße Zusammensetzung 60 bis 98 Gew. % Avarol und 2 bis 40 Gew.% an einer Mischung aus weiteren Inhaltsstoffen, insbesondere antioxidativen Verbindungen der Gruppe der Sesquiterpenderivate. Bevorzugt weist die erfindungsgemäß hergestellte Zusammensetzung 65 bis 95 % Avarol, insbesondere 65 bis 90%, und ganz besonders 70 bis 85 % Avarol und als Rest die genannten Verbindungen, insbesondere antioxidativen Verbindungen auf.
Hierbei handelt es sich insbesondere um- substituierte Sesquiterpenderivate wie (Thio)-Furan- Derivate wie z. B. Thiofurodysinin- und acylierte Derivate, hiervon, deren Aktivität gegenüber DPPH und insofern antioxidative Wirkung im erfindungsgemäßen Extrakt in den nachfolgenden Beispielen aufgezeigt wurde. Daneben können auch Abkömmlinge von Avarol und Avaron enthalten sein, wie 2' oder 5'- acylierte oder 3' oder 4'- Aminoalkylierte, insbesondere Aminomethyl-Derivate, hydrierte Analoga, Stereoisomere hiervon vorhanden sein. Diese Sesquiterpenderivate sind an sich bekannt. Die Sesquiterpenderivate liegen in unterschiedlichen Anteilen jeweils vor.
Es hat sich gezeigt, dass ein derartiger Extrakt pharmakologisch besser wirkt als bisherige Extrakte, die auf die oben beschriebene Weise erhalten wurden, wie in den nachfolgenden Beispielen noch näher erläutert wird.
Der erfindungsgemäße Avarol- Zusammensetzung kann, falls gewünscht, in einem weiteren einfachen, aber effektiven Verfahrensschritt zu hochreinem Avarol umgesetzt werden. Hochreines Avarol ist insbesondere erwünscht für die Synthese von pharmakologisch wirksamen Stoffen wie weiteren Antitumormitteln aus Avarol, das zu diesem Zwecke rein vorliegen muss. Hierzu wird die gewonnene feste Avarol -haltige Zusammensetzung in einem polaren organischen Lösungsmittel gelöst und sodann ein unpolares organisches Lösungsmittel in einer Menge hinzufügt, sodass hochreines Avarol aus der unpolaren Phase auskristallisiert.
Als polares Lösungsmittel eignen sich vor allem die oben beschriebenen Hydroxylgruppen- haltigen Lösungsmittel, insbesondere Ethanol, Methanol, Propanol, Isopropanol oder Mischungen hiervon oder mit Wasser, insbesondere Ethanol, Methanol, Mischungen hiervon oder mit Wasser wie beschrieben. Als unpolares Lösungsmittel eignen sich vor allem C₄C₁₀- aliphatische oder cycloaliphatische oder aromatische Kohlenwasserstoffe. Hierzu gehören vor allem n- Hexan, Cyclohexan, Petrolether (Siedepunkt 40-70° C) oder Mischungen hiervon. Das Mengenverhältnis der Lösungsmittel polar: unpolar beträgt je nach eingesetzter Apparatur 1-10 : 1 oder 1:1-10 und ist derart, dass reines Avarol aus der unpolaren Phase auskristallisiert. Hieraus kann Avarol durch bekannte Maßnahmen wie Filtration oder Zentrifugation abgetrennt werden.
Darüber hinaus ist es auch möglich, dass man insbesondere das so erhaltene Avarol oder die Avarol- haltige Zusammensetzung in einem weiteren Schritt in einem Lösungsmittel, ausgewählt aus polaren, Hydroxylgruppen- haltigen Lösungsmittel wie oben beschrieben, insbesondere Ethanol, Methanol, Propanol, Isopropanol oder Mischungen hiervon oder mit Wasser, insbesondere Ethanol, Methanol, Mischungen hiervon oder mit Wasser, löst , so dass die Avarol Konzentration 10 bis 70 %, bevorzugt 10 bis 50 %, bezogen auf die Menge = Gewicht beträgt, und dann pro Mol Avarol 0,5 bis 1,5 Mol eines Oxidationsmittels, ausgewählt aus Cer(IV)-sulfat, Cer(IV)-ammoniumsulfat, Jodate, Perjodate, Vanadate oder Bleidioxid hinzusetzt und anschließend die gebildete Chinonverbindung (Avaron) auf übliche Weise durch Ausfällen gewinnt.
Besonders bevorzugt werden Cer-(IV)- Verbindungen eingesetzt.
Somit können auf einfache und kostengünstige Weise reines Avarol und Avaron gewonnen werden zur weiteren Verwendung z.B. zur effektiven Synthese von aktiven Wirkstoffen wie Antitumormittel, die bisher nur auf aufwendige Weise erhalten werden konnten.

### Vorteile / Anwendungen des Gegenstandes der Erfindung

Somit ist es erstmals möglich, auf aufwendige chromatographische Reinigungsverfahren bei der Gewinnung von Avarol und Avaron zu verzichten und die gewünschten Produkte auf einfache Weise kostengünstig ohne großen technischen Aufwand in hohen Ausbeuten von 1 bis 30 %, bezogen auf die Ausgangsmaterialien wie Schwämme bzw. Biozellmasse oder Extrakt zu gewinnen. Beispielsweise werden aus 3 kg Schwamm Disidea avara 47 g Avarol (90 %ig ≡ 42,3 g reines Avarol = 1,4 % erhalten. Dies entspricht dem 6- fachen der bisher aus Meeresschwämmen gewonnen Avarol - Menge gemäß Stand der Technik, nämlich 2,7 g Avarol aus 1 Kg Schwamm Disidea avara = 0,27 %. Im allgemeinen werden 5 bis 15 mal bessere Ausbeuten erzielt.
Insbesondere kann erfindungsgemäß eine Avarol- Zusammensetzung erhalten werden, die noch bestimmte Antooxidativa, insbesondere aus der chemischen Gruppe der Sesquiterpenderivate aufweist. Dabei wird bevorzugt als Ausgangsmaterial Schwamm oder Schwammbiozellmasse der Art Disidea avara, aber auch Extrakte hieraus wie beschrieben, eingesetzt, die zum beschriebenen Produkt führen. Diese Zusammensetzung hat sich als hoch bzw. besser wirksam zur topischen, oralen, subkutanen, systemischen oder dermatologischen, topisch kosmetischen Behandlung oder Prävention von Proliferationsstörungen bei Menschen oder Tieren erwiesen. Insbesondere ist sie geeignet zur Behandlung/ Prävention von Schuppenflechte (Psoriasis) , Hauttumoren oder Tumoren des Gastrointestinaltraktes oder der ableitenden Harnwege sowie viralen Erkrankungen wie AIDS, CMV, HSV, Entzündungen wie Arthritis, Rheumatoide Erkrankungen, Dermatitis, Erkrankungen des Bewegungsapparates. Dazu wird die Avarol- Zusammensetzung als pharmazeutisches oder dermatologisches oder kosmetisch wirksames Mittel in Form einer Salbe, Creme, Lotion, Lösung, Gel, liposomalen oder nanopartikulären Zusammensetzung, Tablette, Globuli oder Kapsel, mit einem oder mehreren pharmazeutisch oder kosmetisch üblichen Zusatz- oder Hilfsstoffen formuliert. Dabei kann für ein kosmetisches Mittel eine gegenüber dem pharmazeutisch wirksamen Mittel reduzierte Wirkdosis aufweisen. Es kann hier auch eine Kombination mit anderen Wirkstoffen wie z. B. Naturextrakten (Kamille, Hamamelis, Calendula etc) vorliegen.
Zur Herstellung einer flüssigen Verabreichungsform kann das feste Produkt gelöst werden im gewünschten Medium, wie in Wasser, Alkohol, insbesondere Ethanol, Propanol, Ethylenglykol, Propylenglykol oder Mischungen hiervon, physiologischer Kochsalzlösung oder anderen geeigneten pharmazeutisch zulässigen Trägern oder Mischungen hiervon, wobei die jeweils dann gewünschte Konzentration eingestellt werden kann. Mit flüssigen Trägern werden Gele, Injektionen und Lösungen erhalten. Bei Einsatz von geeigneten Fettphasen können auf übliche Weise Cremes oder Salben hergestellt werden. Als Fettphasen eignen sich z. B. natürliche Öle oder Wachse wie Mandelöl, Pfirsischkernöl, Weizenkeimöl, Sonnenblumen- Jojobaöl oder Olivenöl oder Kohlenwasserstoffe wie flüssige Paraffine, (Paraffin Perliquidum) Isoparaffine, Dioctylcyclohexane, Isohexadecane, oder Squalen, Squalan, insbesondere auch Oleylalkohol, Octyldodecanol (Eutanol® G);Fettsäureester, z.B. Isopropylfettsäureester (Palmitat, Myristat, Isostearat, Oleat),oder ähnliche; ferner Triglyceride wie Caprylic/Capric Triglyceride (Miglyol® 810, 812) , Propylene Glycol Dicaprylate/ Dicaprate (Miglyol® 840); oder Silikonöle und -wachse. Besonders bevorzugte Öl-Komponenten sind flüssige Paraffine und natürliche Öle.
Emulgatoren werden bevorzugt nicht eingesetzt. Falls sie jedoch erwünscht sein sollten, könnten hierfür bekannte und gängige Produkte eingesetzt werden, z.B. W/O- oder O/W- Emulgatoren. Zu den W/O-Emulgatoren gehören z.B. Cetylstearylalkohol, Glycerinmonostearat oder Span ® 85 (Sorbitantrioleat) oder andere pharmazeutisch übliche Sorbitanderivate, wie sie in den gängigen Pharmakopöen zu finden sind, oder analoge Produkte wie Arlacel 85, Span 65 (Sorbitantristearat) Arlacel 65, Propylenglykolmonostearat,
Sorbitanmonooleat, Span 40, Arlacel 40, Span 20 oder andere pharmazeutischtechnologisch übliche Analoga.
Zu geeigneten O/W-Emulgatoren gehören Polyethylenglycole mit geeignetem Ethoxylierungsgrad; wie z.B, wie z.B. Polyoxyethylenglykol-400―monostearat, oleylether, -monolaurat,-sorbitanmonolaurat, -sorbitantristearat).
Zur Herstellung von Salben verwendet man bevorzugt Kohlenwasserstoffe und Wollwachsalkohole.
Zur Herstellung von Kapseln oder Tablette werden hierfür gebräuchliche Hilfsstoffe wie Lactose, Saccharoe, Sorbit, Talkum, Stearinsäure, Magnesiumstearat, Gum arabicum, Kartoffelstärke, Gelatine, PVP, Konservierungsmittel sowie übliche Materialien für Überzüge wie PEG 6000, maisstärke, Zucker Talkum, eingesetzt.
Die Mengen an Fett, ggf. Emulgator, Zusatzstoffen und Tablettierungsverfahren sind allgemein bekannt und z.B. beschrieben in DAB10 oder dem aktuellen Europäischen Arzneibuch.
Gegebenenfalls kann Avarol in geeignete Derivate überführt werden, welche pharmazeutisch verträglich sind und bei Anwendung die aktive Wirkstoffform freisetzen. Hierzu gehören Ester wie Acetate, Propinate, Butyrate, Valerate, Caprylate, Succinate (C-2- und /oder C5), die als solche bekannt und z. B. in der oben genannten US - A 5,082,865 beschrieben sind.
Die Menge des verabreichten Mittels und der Dosierungsplan zur Behandlung eines krankhaften oder vorzubeugenden Zustandes mit dem beschriebenen Mittel hängen von einer Vielzahl von Faktoren ab, einschließlich dem Alter, Gewicht, Geschlecht und medizinischem / kosmetischen Zustand des Patienten, der Schwere der Erkrankung, der Verabreichungsroute und der Häufigkeit der Verabreichung und der jeweiligen verwendeten Verbindung, und kann deshalb weitestgehend schwanken. Bei der Herstellung der Formulierungen können ggf. ein oder mehrere Adjuvantien, die sich für die angegebene Verabreichungsroute eignen, hinzugefügt werden. Wenn per os verabreicht wird, kann das beschriebene Trockenprodukt auch vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykole Polyvinylpyrrolidon und/oder Polyvinylalkohol und dann tablettiert oder verkapselt werden zur einfachen Verabreichung. Formulierungen für die parenterale Verabreichung können in Form von wässrigen oder nicht-wässrigen isotonischen sterilen Injektionslösungen oder Suspensionen vorliegen. Diese Lösungen und Suspensionen können hergestellt werden aus dem erfindungsgemäßen kristallinen Produkt mit einem oder mehreren der Adjuvantien, wie sie genannt wurden für die Verwendung in den Formulierungen für orale Verabreichung. Die Mittel können gelöst sein in Wasser, Polyethylenglykol, Propylenglykol, Ethanol, Maisöl, Baumwollsamenöl, Erdnußöl, Olivenöl, Sesamöl, Benzylalkohol, Natriumchlorid und/oder verschiedenen Puffern ( Stickstoff-/Phosphat-/Sulfat-/Carbonatpuffer ). Bei Wasser/Alkohol als Lösungsmittel entstehen wieder die bereits beschriebenen Gele. Es können auch pH-Regulatoren wie HCl, Phosphorsäurepuffer, NaOH, EDTH-Na, oder NH40H, K2C03 oder andere vorliegen. Ferner können Parfüm-, Farbstoffe, zu denen die genannten Terpene auch gehören oder UV-Filter wie Titandioxid, oder Konsistenzgeber in topisch applizierbaren Produkten enthalten sein.
Ggf. können auch Stabilisatoren und Konservierungsmittel enthalten sein, im allgemeinen kann jedoch darauf verzichtet werden .
Die Menge an Zusatzstoffen beträgt vorzugsweise 0-10, insbesondere 0,1-5 Gewichtsprozent insgesamt.
Insbesondere können entzündliche Zustände / Erkrankungen wie Arthritis, bakterielle und mikrobielle Entzündungen, Psoriasis, rheumatische Erkrankungen sowie degenerative Veränderungen des Bewegungsapparates behandelt oder vorgebeugt werden.
Ferner können auch orale Anwendungen bei viralen Infektionen wie beschrieben erfolgen.
Ganz besonders bevorzugt sind topische Anwendungen in Form von Cremes, Salben Gelen, vor allem der Einsatz von Cremes.
Für eine orale Anwendung eignen sich Gelartige Produkte (Hydro - oder Lipogele) oder vor allem auch Globuli.

### Beispiele

Die Erfindung wird anhand der nachfolgenden Beispiele näher erläutert.

### I. Herstellung von Avarol- Zusammensetzungen, enthaltend 60-98% Avarol

### Beispiel 1

Frisch gesammelter Schwamm Dysidea avara (Feuchtgewicht ca. 300 g) wird mit 500 ml Ethanol (90 %), das 30 g Natriumdithionit enthält, bedeckt und kräftig geschüttelt (Ethanol-Konserve). Hierdurch wird Avarol aus den Schwammzellen gelöst, wobei das Gemisch mehrere Tage stehen gelassen werden kann. Es fällt aus der alkoholischen Lösung durch Zugabe von Wasser aus und wird auf übliche Weise wie Fitration, oder Abnutschen abgetrennt. Das ursprüngliche, einmal extrahierte Ausgangsmaterial (Schwamm) kann erneut mit frischem Ethanol/Dithionit in geringeren Mengen wie oben angegeben behandelt werden und die vereinigten Extrakte z.B. mit Wasser, vorzugsweise enthaltend Dithionit, behandelt und so das gewünschte Produkt erhalten werden.

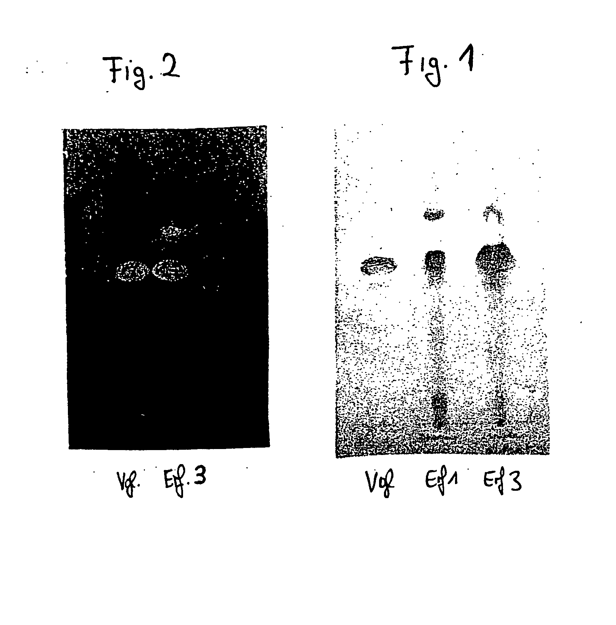

Aus dem DC/ HPLC- Chromatogramm gemäß Fig. 1 (links: reines Avaraol (Vgl.), Mitte und rechts erfindungsgemäße Zusammensetzung (Erf.1 sowie von nachfolgendem Bsp. 3 = Erf.3) und dem NMR der ethanolischen Konservierungslösung ergibt sich ein Gehalt von ca. 80 % bzw. 85 -90 % Avarol und ca. 20 % bzw. 10-15% weiterer Verbindungen, nämlich Antioxidativa wie beschrieben.

### Beispiel 2

Das in Beispiel 1 beschriebene Verfahren wird wiederholt mit der Änderung, das als Lösungs- (Extraktions-) mittel Methanol ( 99%ig, 450 ml) und als Reduktionsmittel Natriumsulfit (50g) verwendet wurden.

### Beispiel 3

Mehrere gemäß Beispiel 1 erhaltene Lösungsmittelextrakte, aus denen mittels der beschriebenen ersten Extraktion Avarol herausgelöst, jedoch noch nicht abgetrennt worden ist, (die vereinigten Extrakte von etwa 300g feuchtem Schwammmaterial ) werden erneut mit Wasser, enthaltend Natriumdithionit (0,01 % in einem Liter Wasser) versetzt. Die Lösung wird trübe und alsbald entsteht ein flockiger Niederschlag. Man lässt vorzugsweise über Nacht im Kühlschrank stehen und filtriert dann ab. Man erhält 2.95 g eines weiß- beigen Pulvers, das auf dem Dünnschichtchromatogramm RP-18 mit Methanol als Laufmittel mit dem Chromatogramm des ursprünglichen ethanolischen Extraktes (Bsp. 1) identisch ist und weitgehend ca. 85 - 90 % Avarol und ca. 10- 15% Antioxidativer Substanzen aufweist. Dies konnte auch im qualitativen Diphenylpicrylhydrazyl (DPPH)-Radikal-Anti-Oxidans-Test wie folgt gezeigt werden:
Der erhaltene Extrakt sowie parallel reines Avarol werden auf Reversed Phase DC Platten der Firma Merck, Darmstadt mit Methanol chromatographiert. Nach der Entwicklung des Chromatogramms wird im kurzwelligen UV betrachtet und die detektierbaren Substanzen markiert. Anschließend wird das Chromatogramm mit 1x10⁻⁶ mol Diphenylpicrylhydrazyl (DPPH) in Methanol detektiert. Durch Entfärbung des violetten Hintergrunds zeigen sich die anti-oxidativ wirksamen Substanzen im erfindungsgemäßen Primärextrakt an. In Fig. 2 ist das Chromatogramm von reinem Avarol (Vgl.), und von der erfindungsgemäßen Avarol -Zusammensetzung gemäß Beispiel 3 (Erf.3) (hier Primärextrakt genannt) zu sehen und zeigt neben Avarol einen Anteil antioxidativer Verbindungen wie beschrieben.

Ein derartiger Extrakt ist insbesondere für die topische Anwendung, z. B. in Cremes etc. zur begleitenden Behandlung von Psoriasis geeignet.

### II. Herstellung von reinem Avarol aus Avarol- haltigen Zusammensetzungen

### Beispiel 4

Aus den vereinigten Extrakten, die gemäß Beispiel 2 mit der Änderung, dass dort Natriumbisulfit und Ethanol eingesetzt wurden, in einem ersten Extraktionsschritt gewonnen wurden, wurden durch Zugabe von Wasser ausgefällt und kristallin gewonnen. Dieses Kristallinat ( 10g) wurde in 150 ml Methanol gelöst und in einen Flüssig- Flüssig-Extraktor gegeben und mit 500 ml Petrolether (40 - 70°C)versetzt. Ggf. kann hier die polare Phase mit Wasser versetzt werden, falls die Phasentrennung nicht einwandfrei ist. Das Avarol geht sodann in die unpolare Phase über, wobei dies durch Erhitzen des Petrolethers in der Vorlage gefördert werden kann. Nach Beendigung lässt man über Nacht stehen. Am nächsten Morgen können die ausgefallenen büschelförmigen, farblosen Kristalle aus der unpolaren Phase abgetrennt werden. Das erhaltene Avarol ist bereits analysenrein. Mp: 152.3°C (unkorr.). Lit. 148-150°C (gemäß Tetrahedron Letters **1974** 3401).

### Beispiel 5

Als Ausgangsmaterial wird ein aus den bisherigen klassischen teerartigen Dysidea avara - mit Essigester gewonnener Extrakt (200 ml) mit aq. NaHCO3-Lösung neutralisiert und am Rotationsverdampfer eingedampft. Das verbleibende dunkelbraune Öl wird mit Acetonitril gelöst und am Rückfluß unter Zugabe von Aktivkohle so lange erhitzt, bis die Lösung nahezu farblos ist. Dann wird filtriert und mit 0.01 % wässr. Natriumdisulfit versetzt. Dabei fällt sofort ein flockiger weißer Niederschlag aus. Man lässt über Nacht im Kühlschrank stehen und saugt dann über eine Nutsche ab. Der gesammelte Niederschlag ist reines Avarol Mp: 152.3°C (unkorr.).

### III. Pharmakologische Beispiele

### 1. Quantitativer Diphenylpicrylhydrazyl (DPPH)-Radikal-Anti-Oxidans-Test

2,5×10⁻⁷ mol Diphenylpicrylhydrazyl (DPPH) in 2,5 ml Methanol werden in eine 1cm-Küvette pipettiert. Die Vergleichsküvette enthält Methanol. In die erste Küvette gibt man 3, 10, 30 und 100µl einer Messlösung, die das Antioxidans Avarol bzw. ein Extrakt gemäß Beispiel 3 vorliegender Erfindung (Nativ - oder Primärextrakt) [1×10⁻⁷ mol in Methanol], enthält und misst den Extinktionsverlauf bei 515nm über 10 Minuten im Doppelstrahl-Spektrophotometer.
Der Verlauf der Reaktionskurven von Avarol und dem Primärextrakt ist zwar innerhalb der Messgenauigkeit des Verfahrens vergleichbar. Normiert man jedoch auf den Avarolgehalt, ist der Primärextrakt der Reinsubstanz mindestens um den Faktor 3 überlegen.

### 2. Wirkung des Extraktes bei Patienten mit Psoriasis

10 wegen progredienter Läsionen stationär behandelte Psoriasis-Patienten erhielten im Doppelblindversuch zur Beurteilung der anti-inflammatorischen Wirkung entweder eine Salbe mit 1% reinem Avarol in Eucerinum anhydricum oder eine Salbe mit dem 1%igen Extrakt gemäß Beispiel 3 vorliegender Erfindung in gleicher Grundlage als einmalige Applikation auf eine mindestens 10 cm² grosse Hautfläche. Mittels eines standardisierten Fragebogens sollten sie die Wirkungen der beiden Behandlungen auf die Rötung ihrer psoriatischen Läsionen beurteilen.
Die Auswertung der Fragebögen ergab, dass der Primärextrakt von 8 der 10 Probanden übereinstimmend als die effektivere Zubereitung beurteilt wurde.

### 3. 5-Lipoxygenase-Test

Granulozyten freiwilliger Spender [5-10 x 10⁶ / ml] wurden in gepufferter physiologischer Kochsalzlösung [pH 7,4] mit Arachidonsäure [40µmol] und Calciumionophor [10µmol] und steigenden Konzentrationen [10nMol bis 10µMol] der Prüfsubstanzen, Avarol bzw. einer Avarol - Zusammensetzung gemäß Beispiel 3, bei 37°C 10 Minuten lang inkubiert. Anschließend wurden die Reaktion durch Zugabe von 1ml Methanol beendet, die Proben zentrifugiert und die Konzentration an Biotransformations-Produkten der 5-Lipoxygenase im Überstand per HPLC im UV bei 280 und 235nm analysiert.
Avarol und die neue Zusammensetzung mit hohem Avarolgehalt, der Dysidea avara-Nativextrakt, hemmten die Bildung von Biotransformationsprodukten der 5-Lipoxygenase. Die IC₅₀- Werte unterschieden sich aber auffällig deutlich. Während für Avarol ein IC₅₀- Wert von 1,3 µM ermittelt wurde, der sich mit Literaturangaben deckt, hemmte die neue Zusammensetzung die Bildung von Produkten der 5-Lipoxygenase bereits mit einem IC₅₀- Wert von 58 nM und war damit überraschend stärker wirksam als Avarol.

## Patentansprüche

1. Verfahren zur Herstellung einer Zusammensetzung, enthaltend Avarol in einer Menge von 60 bis 98%, **dadurch gekennzeichnet, dass** man ein Avarol - haltiges Ausgangsmaterial ein oder mehrmals in einem polaren Hydroxylgruppen - haltigen Lösungsmittel aufnimmt und dieser Mischung ein oder mehrere Reduktionsmittel, ausgewählt aus Alkalisalzen von Dithionit-, -disulfit-, bisulfit-Verbindungen, Selenitsalzen, Zitronensäure, Ascorbinsäure, Glutathion, Phosphite oder Mischungen hiervon in einer Menge von 0,01 bis 20 Gew. %, bezogen auf die Menge an Ausgangsmaterial, hinzufügt und sodann das im Lösungsmittel vorliegende Produkt erhält.

2. Verfahren nach Anspruch 1, **dadurch gekennzeichnet, dass** man als Avarolhaltiges Ausgangsmaterial Meeresschwämme, Schwammzellbiomasse oder gentechnologisch gewonnene Avarol- produzierende Biomasse einsetzt.

3. Verfahren nach einem der Ansprüche 1 oder 2, **dadurch gekennzeichnet, dass** man Natrium- oder Kaliumsalze von Dithionit-, -disulfit-, bisulfit- Verbindungen als Reduktionsmittel einsetzt.

4. Verfahren nach einem der Ansprüche 1 bis 3, **dadurch gekennzeichnet, dass** man eine Umsetzung im Reduktionsmittel - haltigen Lösungsmittel vornimmt und die Konzentration an Reduktionsmittel 3 bis 15 Gew.%, bezogen auf die Menge an eingesetztem Ausgangsmaterial beträgt.

5. Verfahren nach einem der Ansprüche 1 bis 4, **dadurch gekennzeichnet, dass** man eine Umsetzung mit einer Konzentration an Reduktionsmittel von 3 bis 15 Gew. % vornimmt, und den erhaltenen Extrakt abtrennt, das Ausgangsmaterial erneut ein oder mehrmals mit Reduktionsmittel in einer Menge von jeweils 0,01 bis 3 Gew.% mit Lösungsmittel, behandelt und sodann jeweils den Lösungsmittelextrakt mit darin enthaltener Avarol Zusammensetzung vor der folgenden Umsetzung abtrennt und ggf. die gewonnenen Lösungsmittelextrakte vereinigt und die darin enthaltene Avarol - Zusammensetzung gewinnt.

6. Verfahren nach einem der Ansprüche 1 bis 5, **dadurch gekennzeichnet, dass** man die Avarol- Zusammensetzung aus dem(n) Extrakt(en) durch Ausfällen durch ein-oder mehrmalige Zugabe von Wasser oder wässriger Lösung, enthaltend 0,01 bis 10 Gew. % Reduktionsmittel, aus den Lösungsmittelextrakt(en) gewinnt.

7. Verfahren gemäß einem der Ansprüche 1 bis 6, **dadurch gekennzeichnet, dass** man als polares Hydoxylgruppen - haltiges Lösungsmittel einwertige C₁₋₆₋aliphatische, cycloaliphatische oder mit aromatischen Gruppen substituierte C₁₋₆₋Alkohole einsetzt.

8. Verfahren nach Anspruch 7, **dadurch gekennzeichnet, dass** als Lösungsmittel einwertige Alkohole, ausgewählt aus Ethanol, Methanol, Propanol, Isopropanol, Butanol, Pentanol, Hexanol oder Mischungen hiervon oder mit Wasser verwendet werden.

9. Verfahren nach einem der Ansprüche 1 bis 8, **dadurch gekennzeichnet, dass** man dem Avarol enthaltenden Extrakt Wasser zufügt , sodann die Avarol - Zusammensetzung in fester Form gewinnt, diese in einem polaren organischen Hydroxylgruppen - haltigen Lösungsmittel löst und sodann ein unpolares organisches Lösungsmittel in einer Menge hinzufügt, sodass hochreines Avarol aus der unpolaren Phase auskristallisiert.

10. Verfahren gemäß Anspruch 8, **dadurch gekennzeichnet, dass** das polare Lösungsmittel ausgewählt ist aus der Gruppe, umfassend Ethanol, Methanol oder Mischungen hiervon oder mit Wasser und das unpolare Lösungsmittel ausgewählt ist aus C₄C₁₀- aliphatischen oder cycloaliphatischen oder aromatischen Kohlenwasserstoffen.

11. Verfahren gemäß Anspruch 10, **dadurch gekennzeichnet, dass** das unpolare Lösungsmittel ausgewählt ist aus Hexan, Cyclohexan, Petrolether oder Mischungen hiervon.

12. Verfahren gemäß einem der Ansprüche 9 bis 11, **dadurch gekennzeichnet, dass** man als Ausgangsmaterial Meeresschwämme, Schwammbiozellmasse oder Lösungsmittelextrakte von Meeresschwämmen einsetzt.

13. Verfahren gemäß einem der Ansprüche 1 bis 12, **dadurch gekennzeichnet, dass** die erhaltene Avarol- Zusammensetzung in einem weiteren Schritt in einem Hydroxylgruppen - haltigen Lösungsmittel gelöst wird, so dass die Avarol Konzentration 10 bis 70 % beträgt, und dann pro Mol Avarol 0,5 bis 1,5 Mol eines Oxidationsmittels, ausgewählt aus Cer(IV)-sulfat, Cer(IV)-ammoniumsulfat, Jodate, Perjodate, Vanadate oder Bleidioxid hinzugesetzt werden, und anschließend die gebildete Chinonverbindung (Avaron) durch Ausfällen gewonnen wird.

14. Avarol - Zusammensetzung, umfassend, 65 bis 95 Gew. % Avarol und 5 bis 35 Gew. % an zusatzlichen antioxidativen Verbindungen.

15. Pharmazeutisches oder dermatologisches wirksames Mittel in Form einer Salbe, Creme, Lotion, Gel, liposomalen oder nanopartikulären Zubereitungen, Lösung, Tablette oder Kapsel, enthaltend eine Zusammensetzung gemäß Anspruch 14 sowie einen oder mehrere übliche Zusatz- oder Hilfsstoffe.

16. Kosmetisch wirksames Mittel in Form einer Salbe, Creme, Lotion, Gel, liposomalen oder nanopartikulären Zubereitungen, Lösung, Tablette oder Kapsel, enthaltend eine Zusammensetzung gemäß Anspruch 14 sowie einen oder mehrere übliche Zusatz- oder Hilfsstoffe.

17. Topisch wirksames Mittel gemäß Anspruch 15 oder 16, **dadurch gekennzeichnet, dass** es in Form einer Creme, Salbe oder Gel vorliegt.

18. Verwendung einer Avarol- Zusammensetzung gemäß Anspruch 14, 15 oder 17 oder einer Avaron- Zusammensetzung, hergestellt gemäß Anspruch 13 zur Herstellung eines Mittels zur oralen, subkutanen, systemischen oder topisch dermatologischen Behandlung oder Prävention von Proliferationsstörungen bei Menschen oder Tieren.

19. Verwendung gemäß Anspruch 18, **dadurch gekennzeichnet, dass** Psoriasis, Hauttumoren oder Tumoren des Gastrointestinalen Traktes oder der ableitenden Harnwege, virale Erkrankungen, Entzündungen, Dermatitis, degenerative Veränderungen des Bewegungsapparates behandelt oder vorgebeugt werden.

20. Verwendung einer Avarol- Zusammensetzung gemäß Anspruch 14, oder eines Mittels gemäß Anspruch 16 oder 17 oder einer Avaron- Zusammensetzung, hergestellt gemäß Anspruch 13, zur Herstellung einer Zusammensetzung zur topischen kosmetischen Anwendung bei Proliferationsstörungen bei Menschen oder Tieren.

## Claims

1. Process for the preparation of a composition containing avarol in a quantity from 60 to 98 %, **characterised in that** an avarol-containing starting material is taken up one or more times in a solvent containing polar hydroxyl groups, and one or more reducing agents selected from alkali-metal salts of dithionite, disulphite and bisulphite compounds, selenite salts, citric acid, ascorbic acid, glutathione, phosphite or mixtures thereof are added to this mixture in a quantity from 0.01 to 20 % by weight, based on the quantity of the starting material, the product present in the solvent then being obtained.

2. Process according to claim 1, **characterised in that** sea sponges, sponge cell biomass or avarol-producing biomass obtained by means of genetic engineering are used as the avarol-containing starting material.

3. Process according to either claim 1 or claim 2, **characterised in that** sodium or potassium salts of dithionite, disulphite or bisulphite compounds are used as reducing agents.

4. Process according to any one of claims 1 to 3, **characterised in that** a reaction is carried out in the reducing agent-containing solvent, and the concentration of reducing agent is 3 to 15 % by weight, based on the quantity of starting material used.

5. Process according to any one of claims 1 to 4, **characterised in that** a reaction is carried out at a reducing agent concentration from 3 to 15 % by weight, the obtained extract is separated, the starting material is again treated with solvent one or more times with reducing agent in a respective quantity from 0.01 to 3 % by weight, the respective solvent extract, with the avarol composition contained therein, is then separated prior to the subsequent reaction, and the solvent extracts obtained are optionally combined and the avarol composition contained therein is obtained.

6. Process according to any one of claims 1 to 5, **characterised in that** the avarol composition is obtained from the solvent extract(s) by means of precipitation, by adding water or aqueous solution, containing 0.01 to 10 % by weight of the reducing agent, one or more times.

7. Process according to any one of claims 1 to 6, **characterised in that** monohydric C₁₋₆ aliphatic or cycloaliphatic alcohols, or C₁₋₆ alcohols substituted with aromatic groups are used as the solvent containing polar hydroxyl groups.

8. Process according to claim 7, **characterised in that** monohydric alcohols selected from ethanol, methanol, propanol, isopropanol, butanol, pentanol, hexanol or mixtures thereof, or mixed with water are used as the solvent.

9. Process according to any one of claims 1 to 8, **characterised in that** water is added to the avarol-containing extract, the avarol composition is then obtained in solid form and dissolved in a polar organic solvent containing hydroxyl groups, and a nonpolar organic solvent is then added in a quantity such that high-purity avarol crystallises out from the nonpolar phase.

10. Process according to claim 8, **characterised in that** the polar solvent is selected from the group comprising ethanol, methanol or mixtures thereof, or mixed with water, and the nonpolar solvent is selected from C₄ C₁₀ aliphatic or cycloaliphatic or aromatic hydrocarbons.

11. Process according to claim 10, **characterised in that** the nonpolar solvent is selected from hexane, cyclohexane, petroleum ether or mixtures thereof.

12. Process according to any one of claims 9 to 11, **characterised in that** sea sponges, sponge cell biomass or solvent extracts of sea sponges are used as the starting material.

13. Process according to any one of claims 1 to 12, **characterised in that**, in a further step, the avarol composition obtained is dissolved in a solvent containing hydroxyl groups, so that the avarol concentration is 10 to 70 %, 0.5 to 1.5 mol of an oxidising agent selected from cerium(IV) sulphate, cerium(IV) ammonium sulphate, iodates, periodates, vanadates, or lead dioxide are added per mol of avarol, and the quinone compound (avaron) that is formed is then obtained by means of precipitation.

14. Avarol composition comprising 65 to 95 % by weight of avarol and 5 to 35 % by weight of additional antioxidative compounds.

15. Pharmaceutical or dermatological active agent in the form of an ointment, cream, lotion, gel, liposomal or nanoparticulate preparations, solution, tablet or capsule, containing a composition according to claim 14 and one or more conventional additives or auxiliary agents.

16. Cosmetically active agent in the form of an ointment, cream, lotion, gel, liposomal or nanoparticulate preparations, solution, tablet or capsule, containing a composition according to claim 14 and one or more conventional additives or auxiliary agents.

17. Topically active agent according to either claim 15 or claim 16, **characterised in that** it is in the form of a cream, ointment or gel.

18. Use of an avarol composition according to claim 14, claim 15 or claim 17 or of an avaron composition prepared according to claim 13, for the preparation of an agent for the oral, subcutaneous, systemic or topically dermatological treatment or prevention of proliferation disturbances in human beings or animals.

19. Use according to claim 18, **characterised in that** psoriasis, skin tumours or tumours of the gastrointestinal tract or the urinary passages, viral diseases, inflammations, dermatitis and degenerative changes to the locomotor system are treated or prevented.

20. Use of an avarol composition according to claim 14 or of an agent according to either claim 16 or claim 17, or of an avaron composition prepared according to claim 13, for the preparation of a composition for topical cosmetic application in the event of proliferation disturbances in human beings or animals.

## Revendications

1. Procédé de préparation d'une composition, contenant de l'avarol en une quantité de 60 à 98%, **caractérisé en ce que** l'on reprend un matériau de départ contenant l'avarol, une ou plusieurs fois, dans un solvant polaire contenant des groupes hydroxyle et l'on ajoute à ce mélange, un ou plusieurs agents de réduction, choisis parmi des sels alcalins de composés dithionite, disulfite, bisulfite, des sels de sélénite, l'acide citrique, l'acide ascorbique, la glutathione, un phosphite ou leurs mélanges, en une quantité de 0,01 à 20% en poids, sur une base de la quantité de matériau de départ et on obtient le produit dans un solvant.

2. Procédé selon la revendication 1, **caractérisé en ce que** l'on met en oeuvre, comme produit de départ contenant de l'avarol, des éponges marines, la biomasse cellulaire d'éponge ou une biomasse produisant de l'avarol, obtenue par génie génétique.

3. Procédé selon l'une des revendications 1 ou 2, **caractérisé en ce que** l'on met en oeuvre des sels de sodium ou de potassium de composés dithionite, disulfite, bisulfite, comme agent de réduction.

4. Procédé selon l'une des revendications 1 à 3, **caractérisé en ce que** l'on procède à une réaction dans le solvant contenant l'agent de réduction et que la concentration de l'agent de réduction se situe de 3 à 15% en poids, sur base de la quantité du matériau de départ mis en oeuvre.

5. Procédé selon l'une des revendications 1 à 4, **caractérisé en ce que** l'on procède à une réaction avec une concentration de l'agent de réduction allant de 3 à 15% en poids, et l'on sépare l'extrait obtenu, on traite à nouveau le matériau de départ, une ou plusieurs fois, avec l'agent de réduction en une quantité de chaque fois, 0,01 à 3% en poids avec un solvant, et l'on sépare chaque fois, l'extrait par solvant avec la composition contenant l'avarol, avant la réaction suivante, et on purifie le cas échéant, les extraits par solvant obtenus, on obtient la composition contenant l'avarol.

6. Procédé selon l'une des revendications 1 à 5, **caractérisé en ce que** l'on obtient la composition d'avarol à partir du ou des extraits, par précipitation, par addition d'une ou plusieurs fois d'eau ou d'une solution aqueuse, contenant 0,01 à 10% en poids d'agent de réduction.

7. Procédé selon l'une des revendications 1 à 6, **caractérisé en ce que** l'on met en oeuvre comme solvant polaire contenant des groupes hydroxyle, des alcools en C₁₋₆, monovalents, aliphatiques, cycloaliphatique ou substitués par des groupes aromatiques.

8. Procédé selon la revendication 7, **caractérisé en ce que** l'on utilise comme solvant, des alcools monovalents, choisis parmi l'éthanol, le méthanol, le propanol, l'isopropanol, le butanol, le pentanol, l'hexanol ou les mélanges de ceux-ci ou avec de l'eau.

9. Procédé selon l'une des revendications 1 à 8, **caractérisé en ce que** l'on ajoute de l'eau à l'extrait contenant l'avarol, de sorte que l'on obtient la composition d'avarol sous forme solide, celle-ci est dissoute dans un solvant organique polaire contenant des groupes hydroxyle, on y ajoute un solvant organique non polaire en une quantité telle que l'avarol très pur cristallise de la phase non polaire.

10. Procédé selon la revendication 8, **caractérisé en ce que** le solvant polaire est choisi parmi le groupe comprenant l'éthanol, le méthanol ou les mélanges de ceux-ci ou avec de l'eau et que le solvant non polaire est choisi parmi les hydrocarbures en C₄₋₁₀ aliphatiques ou cycloaliphatiques ou aromatiques.

11. Procédé selon la revendication 10, **caractérisé en ce que** le solvant non polaire est choisi parmi l'hexane, le cyclohexane, l'éther de pétrole ou leurs mélanges.

12. Procédé selon l'une des revendications 9 à 11, **caractérisé en ce que** l'on met en oeuvre comme matériau de départ, des éponges marines, des masses cellulaires d'éponge ou des extraits par solvant d'éponges marines.

13. Procédé selon l'une des revendications 1 à 12, **caractérisé en ce que** la composition contenant de l'avarol est dissoute dans une autre étape, dans un solvant contenant des groupes hydroxyle, de sorte que la concentration en avarol se situe de 10 à 70%, et l'on ajoute par mole d'avarol, 0,5 à 1,5 mole d'un agent d'oxydation, choisi parmi le sulfate de cérium (IV), le sulfate de cérium (IV) et d'ammonium, un iodate, un periodate, un vanadate ou le dioxyde de plomb, et ensuite, le composé quinone (avarone) formé est obtenu par précipitation.

14. Composition d'avarol comprenant 65 à 95% en poids d'avarol et 5 à 35% en poids de composés antioxydants supplémentaires.

15. Agent actif de manière pharmaceutique ou dermatologique, sous forme d'une pommade, d'une crème, d'une lotion, d'un gel, de compositions liposomiques ou nanoparticulaires, d'une solution, d'un comprimé ou d'une capsule, contenant une composition selon la revendication 14 ainsi qu'un ou plusieurs additifs ou auxiliaires usuels.

16. Agent actif de manière cosmétique sous forme d'une pommade, d'une crème, d'une lotion, d'un gel, de compositions liposomiques ou nanoparticulaires, d'une solution, d'un comprimé ou d'une capsule, contenant une composition selon la revendication 14 ainsi qu'un ou plusieurs additifs ou auxiliaires usuels.

17. Agent actif de manière topique selon la revendication 15 ou 16, **caractérisé en ce qu'**il se présente sous la forme d'une crème, d'une pommade ou d'un gel.

18. Utilisation d'une composition d'avarol selon la revendication 14, 15 ou 17 ou d'une composition d'avarone, préparée selon la revendication 13, pour la préparation d'un agent pour le traitement ou la prévention orale, sous-cutanée, systémique ou topique de troubles de la prolifération chez l'homme ou les animaux.

19. Utilisation selon la revendication 18, **caractérisée en ce que** l'on traite ou prévient le psoriasis, les tumeurs de la peau ou les tumeurs du tractus gastro-intestinal ou des voies urinaires d'évacuation, les maladies virales, les inflammations, la dermatite, les modifications dégénératives de l'appareil moteur.

20. Utilisation d'une composition d'avarol selon la revendication 14, ou d'un agent selon la revendication 16 ou 17 ou d'une composition d'avarone, préparée selon la revendication 13, pour la préparation d'une composition pour utilisation cosmétique topique lors de troubles de la prolifération chez l'homme ou les animaux.
